# EUROPEAN PATENT APPLICATION

(11) **EP 2 005 888 A2**
(43) Date of publication of application: **24.12.2008**
(21) Application number: 07744290.3
(22) Date of filing: 29.05.2007
(51) Int. Cl.: A61B 5/117, G06T 1/00, G06T 7/00

(54) **IMAGING DEVICE AND AUTHENTICATION DEVICE USING THE SAME**

(30) Priority: 30.05.2006 JP 2006149194
(71) Applicant: Panasonic Corporation, Kadoma-shi Osaka 571-8501 (JP)
(72) Inventor: TSUKAHARA, Shin'ichi c/o Panasonic Corp., IPROC, IP Dev. Center, 7F OBP, Osaka-shi, Osaka 540-6207 (JP); HOSOYA, Masahiko c/o Panasonic Corp., IPROC, IP Dev. Center, 7F OBP, Osaka-shi, Osaka 540-6207 (JP); MIYAZAKI, Keiichi c/o Panasonic Corp., IPROC, IP Dev. Center, 7F OBP, Osaka-shi, Osaka 540-6207 (JP); NAKAIGAWA, Tomoyoshi c/o Panasonic Corp., IPROC, IP Dev. Center, 7F OBP, Osaka-shi, Osaka 540-6207 (JP)
(74) Representative: Pautex Schneider, Nicole Véronique
(86) International application number: PCT/JP2007/060857
(87) International publication number: WO 2007/139084

(57) **Abstract**

Image pick-up device (1) comprises first image pick-up unit (16) for taking an image of a left eye of a subject, second image pick-up unit (17) for taking an image of a right eye of the subject, first lighting unit (11) for illuminating the left eye of the subject, second lighting unit (12) for illuminating the left eye in a direction intersecting an image-taking optical axis of the second image pick-up unit, third lighting unit (13) for illuminating the right eye of the subject, and fourth lighting unit (14) for illuminating the right eye in a direction intersecting an image-taking optical axis of the first image pick-up unit.

## Description

### TECHNICAL FIELD

The present invention relates to an image pick-up device and an authentication apparatus, and more particularly to an image pick-up device for taking an image of a pattern of a retina or an iris for personal identification, and an authentication apparatus using the same.

### BACKGROUND ART

Description is provided first of some techniques used for a conventional image pick-up device and authentication apparatus. An example described herein represents an iris authentication system for identifying individual people.

The iris authentication system for identifying individual people comprises an image pick-up device and an authentication apparatus. The image pick-up device illuminates an eye and its vicinity of a subject by such means as near-infrared lighting, and takes a photographic image of an area including the eye of the subject with a camera. The authentication apparatus extracts an image of iris area out of the photographic image including the eye (hereinafter referred to as "eye image") taken by the image pick-up device, generates a coded authentication information (referred to as "authentication code") as a numerical data representing subtleties of a wrinkle pattern in the iris portion, and executes a comparative verification of the authentication code with another authentication information registered beforehand (referred to as "registered authentication code"). The authentication apparatus authenticates the subject as being an individual registered beforehand when it determines that the codes match with each other as a result of the comparative verification.

In the image pick-up device of this type, there are cases when the subject wears eyeglasses that the illumination light reflected by the lens of the eyeglasses enters into the camera, and this reflected light from the lens overlaps with a part of the obtained eye image (such reflection is referred to hereinafter as "eyeglass reflection"). On the other hand, when the light is projected at an oblique angle for the purpose of reducing the eyeglass reflection, the obliquely illuminated light tends to form a shadow in a marginal area of a hard contact lens if worn by the subject. The obliquely illuminated light may even form a shadow at either the outer angle side or the medial angle side of a naked eye depending on a curvature of the eyeball. When there is a shadow formed in the iris area due to the illumination light as well as such factors as eyeglass reflection, curvature of hard contact lens and also curvature of the eyeball, there arises a missing portion in the iris area required for generating the authentication code, which may hence interfere with the generation of the authentication code.

In consideration of the shape of lenses of ordinary eyeglasses that has a larger curvature near the outer angle side than the medial angle side, image pick-up device for single eye is therefore provided with four light sources L1 through L4, each disposed at the right side, left side, upper right side and upper left side in positions as close as possible to an image-taking optical axis linking between the iris of the subject and the camera, and these light sources L1 through L4 are selectively lit according to the curvature of the lens of either the right eye or the left eye when taking an image of the iris in order to prevent the eyeglass reflection from interfering with the iris image. On the other hand, the two light sources located at the outer angle side of the eye are lit when this image pick-up device is used for taking an image of a subject wearing hard contact lenses (refer to patent document 1 for example).

However, since there was not available any image pick-up device capable of taking images of both eyes of a subject wearing eyeglasses or hard contact lenses, it was necessary to select the light sources to be lit according to a curvature of each lens when taking images of both the right eye and the left eye individually. There is thus a drawback with the conventional image pick-up device that it puts the subject to inconvenience of being forced to make eye positioning at least two times when taking images of both the right and the left eyes for the authentication.

The image pick-up device also has a problem that it generates an inaccurate authentication code or no authentication code at all because of the eyeglass reflection or the shadow formed in the marginal area of the hard contact lens if it fails to attain an adequate matching of the light projecting direction with the curvature of the eyeglasses lens or the hard contact lens. Such authentication code can lead to an erroneous authentication if used for the personal identification.

Another problem associated with the conventional image pick-up device is that when it projects the illumination light to a hard contact lens of a large curvature from one side, it forms a shadow at the other side in a marginal area of the hard contact lens.
[Patent Document 1] Japanese Patent Unexamined Publication, No. 2003-308523

### SUMMARY OF THE INVENTION

The present invention has been made in view of the above problems, and has an object to provide an image pick-up device capable of taking images of both eyes of even a subject wearing eyeglasses or hard contact lenses, while alleviating inconvenience for the subject to make eye positioning, and reducing eyeglass reflection and shadows formed in marginal areas of the hard contact lenses. The invention also provides an authentication apparatus using the image pick-up device.

The image pick-up device of this invention is characterized by having a first image pick-up unit for taking an image of a left eye of a subject, a second image pick-up unit for taking an image of a right eye of the subject, a first lighting unit for illuminating the left eye of the subject, a second lighting unit for illuminating the left eye in a direction intersecting an image-taking optical axis of the second image pick-up unit, a third lighting unit for illuminating the right eye of the subject, and a fourth lighting unit for illuminating the right eye in a direction intersecting an image-taking optical axis of the first image pick-up unit.

Since the image pick-up device of this structure is capable of taking images of both the right eye and the left eye of the subject at the same time while illuminating the both eyes, it does not require the subject to change a position facing the device from one eye to the other, thereby reducing a number of repeating eye positioning and alleviating the inconvenience of the authentication. In addition, the device enables the subject to have images of both the right eye and the left eye taken with the illumination in the optimum directions without making an error in operating the device since the subject is not required to repeat the eye positioning. It is also possible for the image pick-up device to take images of both eyes without causing any dropout even when the subject is wearing hard contact lenses because the eyes are illuminated from both sides to reduce shadows of the hard contact lenses.

In addition, the first lighting unit through the fourth lighting unit may be so designed that quantities of light are individually adjustable.

This structure allows adjustment of the lighting units in a manner so that lighting intensities on surfaces of both the right eye and the left eye become consistent even when light projecting directions of the individual lighting units are different in angle with respect to the image-taking optical axes. The structure also allows adjustment of the lighting units in a manner to balance the quantities of light among them so as to equalize the lighting intensities on both eyes when the eyes are individually illuminated from the both sides at different light projecting directions. Accordingly, this structure can eliminate restrictions in mounting positions of the light sources, thereby allowing a reduction in size of the device.

Moreover, each of the first lighting unit through the fourth lighting unit may comprise a plurality of light-emitting elements, and the quantities of light produced by the first lighting unit through the fourth lighting unit are adjusted individually by changing a number of the light-emitting elements to be lit.

This structure allows adjustment of the lighting units by changing the number of the light-emitting elements in a manner to maintain consistency of the lighting intensities on both eyes.

Furthermore, the first lighting unit through the fourth lighting unit, each comprised of the plurality of light-emitting elements may be lit in a manner to adjust their quantities of light individually by varying a driving current supplied to each of the plurality of light-emitting elements.

This structure allows adjustment of the lighting units by varying the driving currents to the plurality of the light-emitting elements so as to maintain consistency of the lighting intensities on both the right eye and the left eye.

It is also suitable that the first lighting unit through the fourth lighting unit, each comprised of the plurality of light-emitting elements may be lit in a manner to adjust their quantities of light individually by a combination of changing a number of the light-emitting elements to be lit and varying a driving current supplied to each of them.

This method helps make accurate adjustment possible for the lighting intensities on both the right eye and the left eye individually by correcting differences in quantities of light, even if exist among the light-emitting elements, by means of adjusting their driving currents. The method can hence achieve consistency of the lighting intensities on both the right eye and the left eye.

It is also possible to adjust the quantities of light of the first lighting unit and the second lighting unit individually according to angles of their light projecting directions with respect to the image-taking optical axis of the first image pick-up unit, and the quantities of light of the third lighting unit and the fourth lighting unit individually according to angles of their light projecting directions with respect to the image-taking optical axis of the second image pick-up unit.

This method enables adjustment of the quantities of light that vary with the angles of their light projecting directions so as to equalize the lighting intensities over hard contact lenses on both the right eye and the left eye even when the subject is wearing the hard contact lenses.

In another aspect, the image pick-up device may be provided further with a lighting controller for controlling lighting of the first lighting unit through the fourth lighting unit individually, wherein the lighting controller selectively lights up the first, second, third and fourth lighting units one after another in a predetermined lighting pattern.

According to this structure, it becomes possible for the image pick-up device to illuminate the subject, even when wearing eyeglasses or hard contact lenses, from the light sources at the optimum directions toward the eyeglasses or the hard contact lenses by switching the lighting units to be used according to the selected lighting pattern.

The predetermined lighting pattern here can be lighting of any of the second lighting unit, the fourth lighting unit, both the first and the second lighting units, and both the third and the fourth lighting units.

Accordingly, the image pick-up device becomes operative to light the second lighting unit and the fourth lighting unit to the subject wearing eyeglasses, or the first lighting unit and the second lighting unit or the third lighting unit and the fourth lighting unit to the subject of naked eyes or wearing hard contact lenses. The image pick-up device can hence reduce the eyeglass reflection by increasing an inclination of the lighting angle from the light sources to the eyeglasses in this manner. The image pick-up device can also reduce shadows at both the medial angle side and the outer angle side by way of illuminating the naked eyes or to the hard contact lenses from both sides thereof.

The lighting controller may also have a function of controlling individually the quantities of light produced by the light sources of the first lighting unit to the fourth lighting unit in a manner to maintain consistency of the lighting intensities on both the right eye and the left eye according to a combination of the predetermined lighting patterns.

According to this structure, it becomes possible for the image pick-up device to balance the quantities of light among the light sources and maintain the lighting intensities on both eyes even when the subject is wearing eyeglasses or hard contact lenses.

Next, the authentication apparatus of the present invention is characterized by having the image pick-up device discussed above and an authentication unit for authenticating an individual subject as to whether she/he is a person registered beforehand by using eye images taken on both the right eye and the left eye of the subject with the image pick-up device.

This structure enables the authentication unit to obtain from the image pick-up device the eye images of both the right and left eyes, and to carry out the authentication by requiring the subject to make eye positioning only once. As a result, the authentication apparatus can carry out the authentication regardless of whether registered authentication information stored in the authentication unit is a data for any of the right eye, the left eye, or both of them.

Furthermore, the authentication unit can comprise an authentication information generator for generating authentication information respectively representing the right eye and the left eye of the subject from the eye images taken with the image pick-up device, a comparator for comparatively verifying the authentication information of the right eye and the left eye generated by the authentication information generator with authentication information registered beforehand, and an authentication determiner for determining whether the subject is an individual registered beforehand by comparing a degree of matching obtained as a result of the comparative verification by the comparator with a predetermined threshold.

Accordingly, the authentication unit receives the eye images of both the right eye and the left eye from the image pick-up device, thereby enabling it to generate the authentication information for the right eye and the left eye respectively. The authentication unit can thus carry out the authentication regardless of whether the registered authentication information is a data for any of the right eye, the left eye, or both of them.

Alternatively, the authentication determiner may be designed to make the determination as to whether the subject is an individual registered beforehand by using at least one of the authentication information of the right eye and the left eye.

This enables the authentication unit to carry out the authentication regardless of whether the registered authentication information is a data for one of the right eye, the left eye, or both of them.

In addition, the authentication information may be an authentication code generated by encoding an iris area of the eye image.

This enables the authentication unit to carry out authentication of the individual subject by using the authentication code generated by encoding the iris area of the subject.

As described above, the present invention can thus provide the image pick-up device and the authentication apparatus using the image pick-up device, which is capable of taking images of both eyes of even a subject wearing eyeglasses or hard contact lenses, while alleviating inconvenience for the subject to make eye positioning, and reducing the eyeglass reflection and shadows formed in the marginal areas of the hard contact lens.

### BRIEF DESCRIPTION OF DRAWINGS

Fig. 1 is a schematic drawing showing a structure of an authentication apparatus according to an exemplary embodiment of the present invention;
Fig. 2 is a schematic drawing showing a structure of a panel used for guiding positions of eyes in an image pick-up device of this authentication apparatus;
Fig. 3A is a schematic view showing a structure of a light source of the image pick-up device;
Fig. 3B is a schematic view showing a structure of another light source of the image pick-up device;
Fig. 4A is a schematic drawing illustrating a method of adjusting a quantity of light from the light source of the image pick-up device;
Fig. 4B is a schematic drawing illustrating another method of adjusting a quantity of light from the light source of the image pick-up device;
Fig. 5A is a schematic drawing illustrating illumination light projected to an eye of a subject wearing a hard contact lens in the image pick-up device;
Fig. 5B is another schematic drawing illustrating illumination light projected to an eye of the subject wearing a hard contact lens in the image pick-up device;
Fig. 6A is a schematic drawing illustrating illumination light projected to an eye of a subject wearing an eyeglass in the image pick-up device;
Fig. 6B is another schematic drawing illustrating illumination light projected to an eye of the subject wearing an eyeglass in the image pick-up device;
Fig. 7 is a block diagram showing a structure of an authentication unit of the authentication apparatus;
Fig. 8 is a flow chart illustrating an operation of the image pick-up device; and
Fig. 9 is a flow chart illustrating an operation of the authentication apparatus.

### REFERENCE MARKS IN THE DRAWINGS

- 1: Image pick-up device
- 2: Authentication unit
- 3: Authentication apparatus
- 11: Light source (first lighting unit)
- 12: Light source (second lighting unit)
- 13: Light source (third lighting unit)
- 14: Light source (fourth lighting unit)
- 15: Lighting controller
- 16: Camera (first image pick-up unit)
- 17: Camera (second image pick-up unit)
- 18: Image determiner
- 19: Memory
- 20: Transmitter-receiver
- 21: Authentication information generator
- 22: Register
- 23: Comparator
- 24: Data base (DB)
- 25: User register
- 26: Authentication determiner
- 30: and 31 Semi-transparent mirror
- 32: Distance meter
- 33: Eye position determiner
- 34: Panel
- 35 and 36: Window
- 70: Subject
- 71: Left eye
- 72: Right eye
- 73 and 74: Iris
- 81, 82, 83, and 84: Light beam
- 85 and 86: Image-taking optical axis
- 90 and 91: Hard contact lens
- 94 and 95: Eyeglass lens
- 110: Light-emitting element

### DETAILED DESCRIPTION OF PREFERRED EMBODIMENTS

Description is provided hereinafter in detail of an exemplary embodiment of the present invention with reference to the accompanying drawings.

### [Exemplary Embodiment]

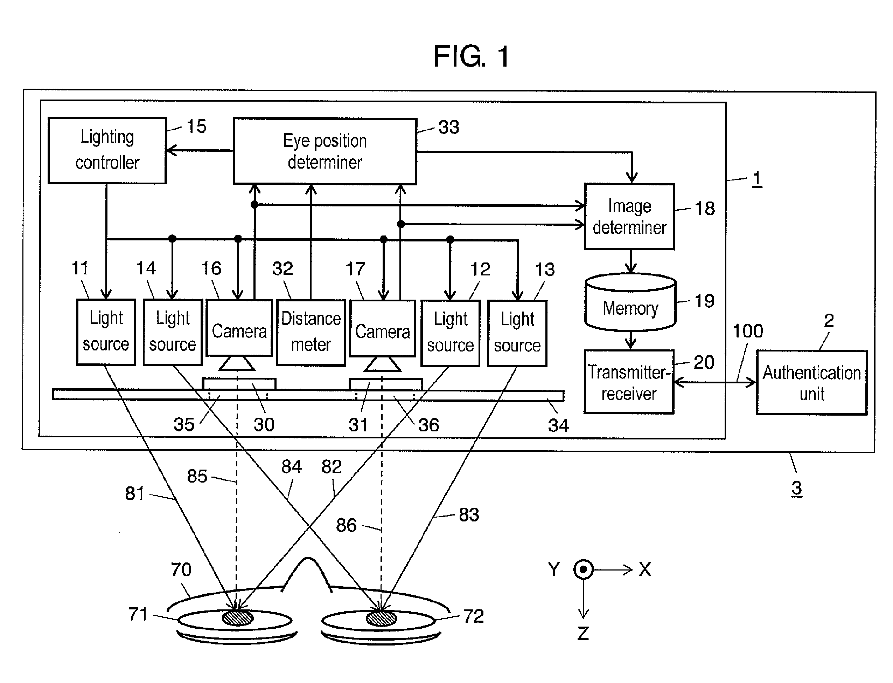

Fig. 1 is a schematic drawing showing a structure of an authentication apparatus according to the exemplary embodiment of this invention.

In Fig. 1, authentication apparatus 3 comprises image pick-up device 1 for taking images of both eyes of subject 70 and authentication unit 2 for carrying out authentication of subject 70 by using the eye images taken by image pick-up device 1.

Description is provided first of image pick-up device 1 by using Fig. 1.

Image pick-up device 1 comprises semi-transparent mirrors 30 and 31 for reflecting both eyes of subject 70 through windows 35 and 36 provided for guiding positions of the both eyes, camera 16 (first image pick-up unit) for taking an image of left eye 71 of subject 70, camera 17 (second image pick-up unit) for taking an image of right eye 72 of subject 70, light source 11 (first lighting unit) for illuminating left eye 71 of subject 70, light source 12 (second lighting unit) for illuminating left eye 71 in a direction intersecting an image-taking optical axis of camera 17, light source 13 (third lighting unit) for illuminating right eye 72 of subject 70, light source 14 (fourth lighting unit) for illuminating right eye 72 in a direction intersecting an image-taking optical axis of camera 16, lighting controller 15 for controlling lighting of light sources 11 to 14, image determiner 18 for evaluating quality of an eye image taken by any of camera 16 and camera 17, memory 19 for storing the eye image, and transmitter-receiver 20 for transmitting and receiving data to/from authentication unit 2.

This structure is capable of taking images of both left eye 71 and right eye 72 for authentication with the eyes positioned only once, rather than requiring subject 70 to change the position twice for each of left eye 71 and right eye 72, like a device of the type designed to take an image of single eye. It therefore alleviates the inconvenience for subject 70. In addition, it enables subject 70 to have images of both the right eye and the left eye taken with illumination in the optimum directions without making an error in operating the device since the subject is not required to repeat positioning of the eyes.

It is also possible for this image pick-up device to equalize the lighting intensities over hard contact lenses or naked eyes having a certain degree of curvature and reduce irregularities of the illumination by way of projecting the illumination lights to the lenses or the eyes from both sides thereof. By virtue of this feature, the device can successfully take images of the both eyes of subject 70 even when wearing eyeglasses or hard contact lenses, while avoiding eyeglass reflection or shadows liable to appear in marginal areas of the hard contact lenses.

Cameras 16 and 17 are so positioned that their image-taking optical axes 85 and 86 point toward left eye 71 and right eye 72 respectively of subject 70, so as to enable them to take images of both left eye 71 and right eye 72 at once.

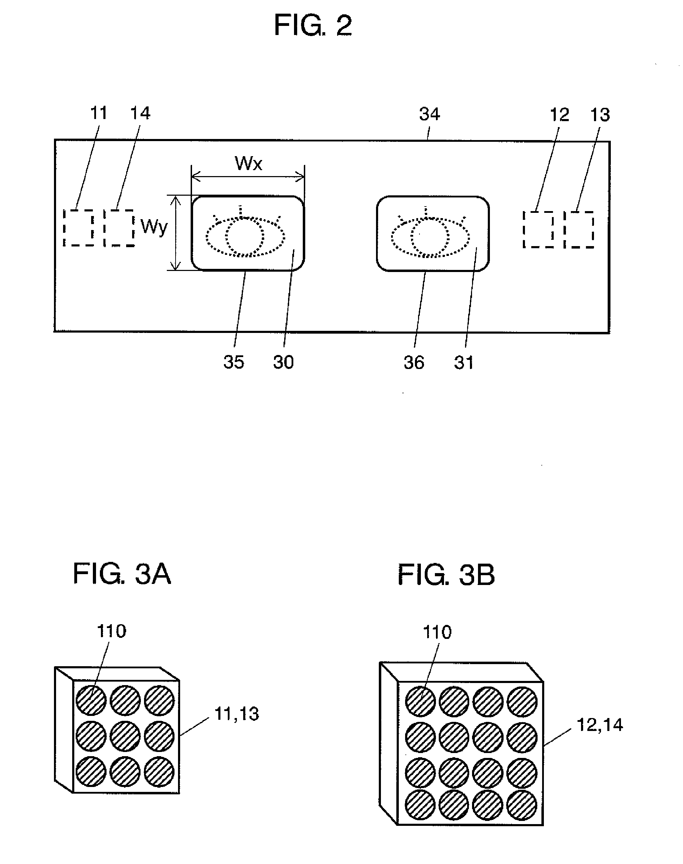

As shown in Fig. 2, cameras 16 and 17 take the images of both eyes of subject 70 from behind semi-transparent mirrors 30 and 31 respectively. In this embodiment, the semi-transparent mirrors are a type that reflects a part of incident light while allowing the rest to penetrate through it.

Image pick-up device 1 can give reflecting images of the both eyes of subject 70 individually on the surfaces of semi-transparent mirrors 30 and 31 through windows 35 and 36 opened in a cover of panel 34. Windows 35 and 36 have a size of "Wy" in height by "Wx" in width, and these dimensions are determined by a size, etc. of image pick-up elements (not shown) employed in cameras 16 and 17. Some of the determinants of the window size are, for instance, an angle of view for taking images, a number of pixels necessary for the authentication, and the like factors as given by the image pick-up element. Subject 70 can align her/his left eye 71 and right eye 72 in their respective positions along the horizontal direction (i.e., X axis) and the vertical direction (i.e., Y axis) while observing the reflection of the eyes on semi-transparent mirrors 30 and 31 through these windows 35 and 36. The positions of the eyes are also guided in the direction of camera-to-subject distance (i.e., Z axis), which will be described later.

Next, light sources 11 and 12 are disposed in a manner to illuminate left eye 71 with their light beams 81 and 82. Here, light source 12 is so positioned that it illuminates left eye 71 in a direction intersecting the image-taking optical axis of camera 17. Likewise, light sources 13 and 14 are disposed in a manner to illuminate right eye 72 with their light beams 83 and 84. Light source 14 is also positioned so that it illuminates right eye 72 in a direction intersecting the image-taking optical axis of camera 16.

Light sources 11 and 12 are arranged generally horizontally along the direction of X axis with a certain variation in their disposed positions. Similarly, light sources 13 and 14 are arranged generally horizontally along the direction of X axis. It is also appropriate that the positions of the light sources are shifted in the direction of Y axis in order to eliminate an effect of shadows formed by eyelashes and the like of the subject. For instance, the illumination lights may be projected to left eye 71 and right eye 72 from a direction slightly lower than the line of sight of subject 70.

Referring now to Fig. 3A and Fig. 3B, description is provided of a structure of light sources 11 to 14. Fig. 3A and Fig. 3B are schematic views showing the structures of different light sources of image pick-up device 1.

Each of light sources 11 and 13 comprises a plurality of light-emitting elements 110 for generating near-infrared light disposed into a matrix form, and there are 9 pieces of light-emitting elements 110 in this exemplary embodiment, as shown in Fig. 3A. Similarly, each of light sources 12 and 14 comprises a plurality of light-emitting elements 110 for generating near-infrared light disposed into another form of matrix, and there are 16 pieces of light-emitting elements 110 here as shown in Fig. 3B.

As discussed, light sources 12 and 14 are each provided with a larger number of light-emitting elements 110 as compared to light sources 11 and 13 so as to make them adaptable for illuminating any of the subjects with naked eyes, wearing hard contact lenses or eyeglasses by controlling lighting of the individual light-emitting elements 110. This enables adjustment of the light sources in a manner to maintain consistency of the lighting intensities on the surfaces of both eyes of subject 70 regardless of whether the eyes are illuminated from one side or two sides. Details of the two-side illumination and the one-side illumination will be described later. Specific methods of adjusting the quantities of light of light sources 11 to 14 will also be described later.

Next, description is provided of a method of guiding the positions of the eyes in the direction of camera-to-subject distance (i.e., Z axis).

Distance meter 32 projects a light pulse from a light projector to an area of a face such as a forehead of subject 70. The light pulse is reflected on the forehead and received in an optical detector. Distance meter 32 measures a distance between the forehead of subject 70 and image pick-up device 1 (in the direction of Z axis) by computing it from a difference in time between the projected pulse and the received pulse, i.e., a time for the light pulse to make a round trip, and the velocity of light, and converts it into the camera-to-subject distance at the position of the eyes. Image pick-up device 1 uses a voice or a display to guide subject 70 according to the measured distance (in the direction of Z axis) in a manner to bring the eyes of subject 70 to the proper camera-to-subject distance along the Z axis to image pick-up device 1. The camera-to-subject distance may be measured by other methods such as the triangulation technique and a method of measuring a distance between two eyes of subject 70 caught with two cameras and converting it into the camera-to-subject distance.

Image pick-up device 1 thus guides the both eyes of subject 70 to the positions of taking the images (in the directions of X axis, Y axis and Z axis) by means of this distance meter 32 and semi-transparent mirrors 30 and 31 for reflecting the eyes through windows 35 and 36.

Eye position determiner 33 determines based on the images taken by cameras 16 and 17 as to whether left eye 71 and right eye 72 of subject 70 are within their respective angles of view for taking images. At the same time, eye position determiner 33 determines whether the positions of the eyes of subject 70 are within an allowable range of focal point based on the camera-to-subject distance measured by distance meter 32. When eye position determiner 33 determines that "the eyes are within the allowable range of focal point", it outputs a result of the determination indicating "the camera-to-subject distance is OK" to lighting controller 15. On the other hand, when eye position determiner 33 determines that "the eyes are out of the allowable range of focal point", it outputs a result of this determination indicating "the camera-to-subject distance is NG" to lighting controller 15.

Furthermore, eye position determiner 33 outputs a result of determination indicating "the eye position is OK" to image determiner 18 when it determines that "the eyes are caught inside the angles of view for taking images" and "the eyes are within the allowable range of focal point". On the contrary, eye position determiner 33 outputs a result of determination indicating "the eye position is NG" to image determiner 18 when it determines that "the eyes are not caught in the angles of view for taking images" or "the eyes are out of the allowable range of focal point".

When lighting controller 15 receives the result of determination indicating "the camera-to-subject distance is OK" from eye position determiner 33, it cause to illuminate the eyes of subject 70. Lighting controller 15 controls lighting-up of light sources 11 to 14 according to a condition of the subject among those of naked eyes, wearing hard contact lenses, wearing eyeglasses, and the like. In addition, lighting controller 15 adjusts the quantities of light of the individual light sources 11 to 14 according to a combination thereof.

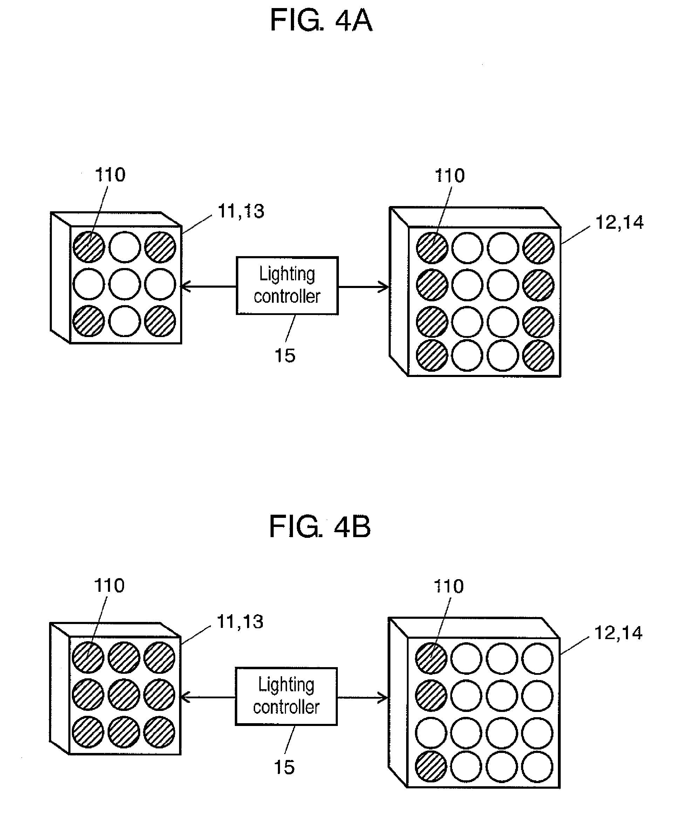

Here, lighting controller 15 adjusts the quantities of light of light sources 11 to 14 in a manner, which is described hereinafter with reference to Fig. 4A and Fig. 4B. Fig. 4A and Fig. 4B are schematic drawings illustrating methods of adjusting the quantities of light produced by light sources 11 to 14 of image pick-up device 1.

Fig. 4A is a drawing illustrating how the quantity of light is adjusted for illuminating the subject of naked eyes or wearing hard contact lenses.

As shown in Fig. 4A, lighting controller 15 controls the light sources so as to illuminate the eyes from the two sides in order to produce the illumination suitable for the naked eyes and the hard contact lenses. The illumination projected by the light sources from the two sides can reduce a shadow liable to form at the medial angle side of left eye 71 due to light source 11 illuminated from the outer angle side even when subject 70 is wearing hard contact lenses of a large curvature. Likewise, this illumination can also reduce a shadow liable to form at the medial angle side of right eye 72 due to light source 13 illuminated from the outer angle side. It also has the effect of reducing shadows by the naked eyes although their curvatures are not so large.

During the two-side illumination, lighting controller 15 lights 5 pieces of light-emitting elements 110 for each of light sources 11 and 13, and 18 pieces of light-emitting elements 110 for each of light sources 12 and 14, as shown in Fig. 4A. Lighting controller 15 adjusts the quantities of light of the light sources in this manner by controlling a number of light-emitting elements 110 to be lit according to light projecting angles of the individual light sources since lighting intensities on the surfaces of the eyes of subject 70 vary depending on the light projecting angles. Accordingly, lighting controller 15 can adjust the lighting intensities obtained by the individual light sources 11 and 12 to an equal value on the eye. Likewise, lighting controller 15 can also adjust the lighting intensities obtained by the individual light sources 13 and 14 to an equal value on the eye, so as to reduce irregularities of the illumination over the surfaces of the eyes or the hard contact lenses.

Alternatively, lighting controller 15 may be so designed as to adjust the quantities of light of the light sources by controlling driving currents to illuminate light-emitting elements 110. For example, lighting controller 15 can change the driving currents by controlling an on-and-off duty ratio of pulse waves, values of electric currents and the like.

As discussed, this method is adapted to adjust the quantities of light of the individual light sources by controlling the number of light-emitting elements 110 to be lit, the driving currents, or a combination of both according to their light projecting angles, thereby increasing a degree of flexibility in positioning the light sources on the panel.

Fig. 4B, on the other hand, is a schematic drawing illustrating how the quantity of light is adjusted to the subject wearing eyeglasses.

As shown in Fig. 4B, lighting controller 15 provides one-side illumination to the subject wearing eyeglasses. Since the one-side illumination is provided by light sources 12 and 14 at a large projecting angle, it can reduce eyeglass reflection on the glass lenses even if the subject moves the face to the right or the left to a certain degree.

In this mode of the one-side illumination, lighting controller 15 turns off all light-emitting elements 110 of light sources 11 and 13, whereas it lights 13 pieces of light-emitting elements 110 for each of light sources 12 and 14 to produce lighting intensities of the same level as that of the two-side illumination on the eyes of subject 70, as shown in Fig. 4B. In this manner, lighting controller 15 can ensure the lighting intensities of the same level as the two-side illumination on the eyes of subject 70 even when the eyes are illuminated from one side.

It is also appropriate to correct variations in quantities of light produced by the individual light-emitting elements 110 by adjusting the quantities of light of light-emitting element 110 with the driving currents in the same manner as in the case of two-side illumination. In a case that all 16 pieces of light-emitting elements 110 are lit, for instance, their driving currents can be adjusted to obtain a total quantity of light equivalent to that when 13 light-emitting elements 110 are illuminated.

As discussed, this method can adjust the lighting intensities on the eyes even though light projecting directions from the light sources are tilted in angle away from the image-taking optical axis, thereby increasing a degree of flexibility in positioning the light sources. This can make possible a reduction in size of the device by putting some contrivances into positional arrangement of the light sources.

Lighting controller 15 is designed to switch the mode of illumination from one for hard contact lenses or naked eyes to another for eyeglasses. This helps reduce the shadows formed in the marginal areas of the hard contact lenses by virtue of the two-side illumination even when subject 70 is wearing the hard contact lenses of a large curvature. This also helps reduce the eyeglass reflection with the one-side illumination by using the light sources of the large projecting angle to the image-taking optical axis when subject 70 is wearing eyeglasses having a small curvature.

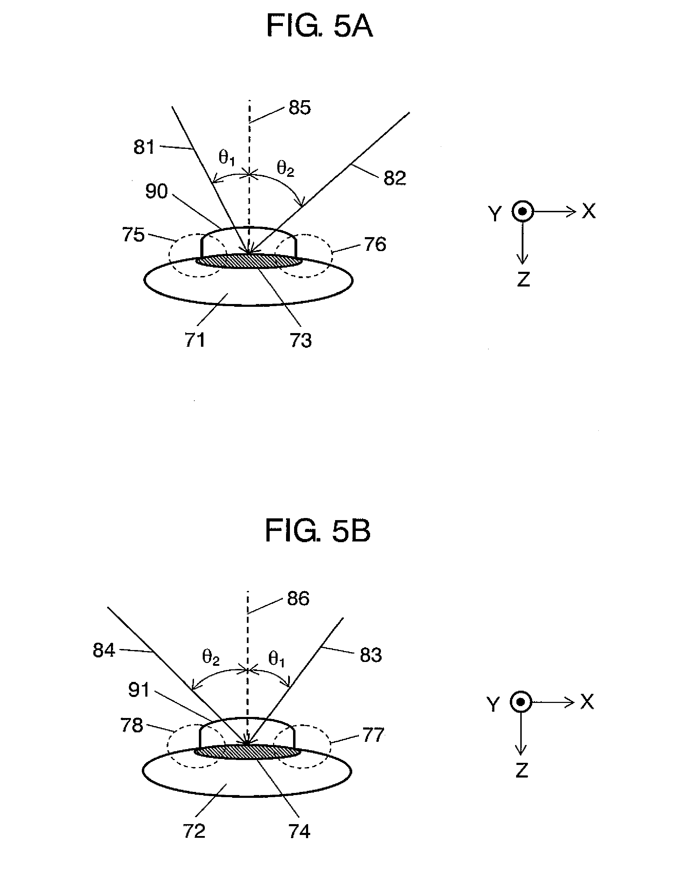

Referring to Fig. 5A and Fig. 5B, detailed description is provided next of the illumination lighting when the subject is wearing hard contact lenses. Fig. 5A and Fig. 5B are schematic drawings illustrating illumination light projected to eyes of the subject wearing the hard contact lenses.

Lighting controller 15 lights light sources 11, 12, 13 and 14 when confronting any of naked eyes and hard contact lenses to illuminate left eye 71 and right eye 72.

Light source 11 projects light beam 81 to iris 73 of left eye 71 of subject 70 wearing hard contact lens 90 at angle θ1 with respect to image-taking optical axis 85 of camera 16 as shown in Fig. 5A. On the other hand, light source 12 projects light beam 82 to iris 73 at angle θ2 with respect to image-taking optical axis 85 of camera 16. In this instance, light beams 81 and 82 of light sources 11 and 12 intersect at iris 73. These light beams 81 and 82 have the relation of angle θ2 > angle θ1. As a result, light beam 81 of light source 11 can reduce a shadow liable to form in an edge area at outer angle side 75 of the eye due to the curvature of hard contact lens 90 and by light beam 82 of light source 12. Likewise, light source 12 can reduce a shadow liable to form at medial angle side 76 of the eye by light source 11.

There is also a case when angle θ2 of light source 12 is increased beyond a predetermined value that the marginal area around hard contact lens 90 is overly brightened due to propagation of the light to the edge area at outer angle side 75 of hard contact lens 90. If the marginal area of hard contact lens 90 is brightened, the marginal area of hard contact lens 90 is identified incorrectly as a pattern of iris 73, and the authentication unit may generate an inaccurate code when it generates an authentication code. When this is the case, the intense brightness in the marginal area can be reduced by adjustment to weaken the quantity of light from light source 12. The intense brightness in the marginal area attributed to light source 11 can also be reduced in the similar manner by weakening the quantity of light from light source 11.

Similarly, as shown in Fig. 5B, light source 13 projects light beam 83 to iris 74 of right eye 72 of subject 70 wearing hard contact lens 91 at angle θ1 with respect to image-taking optical axis 86 of camera 17. On the other hand, light source 14 projects light beam 84 to iris 74 at angle θ2 with respect to image-taking optical axis 86 of camera 17. In this instance here, light beams 83 and 84 of light sources 13 and 14 intersect at iris 74. The light beams 81 and 82 also have the relation of angle θ2 > angle θ1. As a result, light beam 83 of light source 13 can reduce a shadow liable to form in an edge area at outer angle side 77 of the eye due to the curvature of hard contact lens 91 and by light beam 84 of light source 14. Likewise, light source 14 can reduce a shadow liable to form at medial angle side 78 of the eye by light source 13.

There is also a case when angle θ2 of light source 14 is increased beyond the predetermined value that the marginal area around hard contact lens 91 is overly brightened due to propagation of the light to the edge area at outer angle side 77 of hard contact lens 91, and this intense brightness in the marginal area can be reduced by adjustment to weaken the quantity of light from light source 14. The intense brightness in the marginal area attributed to light source 13 can also be reduced in the similar manner by weakening the quantity of light from light source 13.

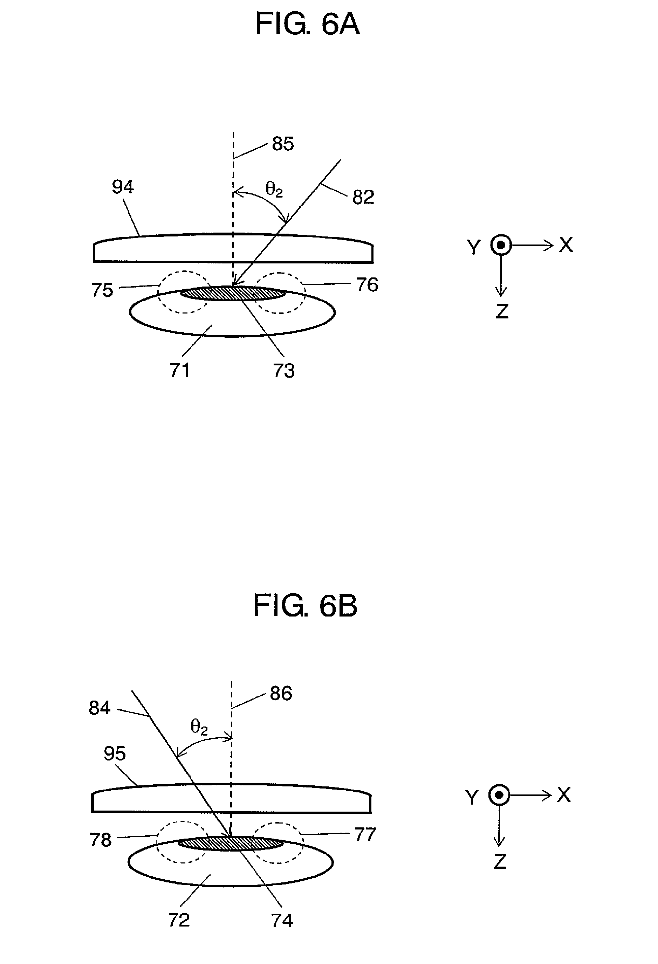

Description is provided next of the illumination lighting when the subject is wearing eyeglasses by referring to Fig. 6A and Fig. 6B. Fig. 6A and Fig. 6B are schematic drawings illustrating illumination light projected to eyes of the subject wearing eyeglasses.

Lighting controller 15 lights up light sources 12 and 14 to illuminate left eye 71 and right eye 72 respectively of the subject wearing eyeglasses.

Light source 12 projects light beam 82 to iris 73 of left eye 71 through eyeglass lens 94 worn by the subject in a direction intersecting with image-taking optical axis 85 of camera 16 at angle θ2 as shown in Fig. 6A.

As stated, light source 12 having a large inclination of the projecting angle to image-taking optical axis 85 is used for left eye 71 when illuminating the subject wearing eyeglasses, so as to prevent light beam 82 of light source 12 from entering camera 16 directly and to reduce the eyeglass reflection even if subject 70 moves the face slightly to a different orientation.

Light source 14 projects light beam 84 in the same manner to iris 74 of right eye 72 through eyeglass lens 95 worn by the subject in a direction intersecting with image-taking optical axis 86 at angle θ2 as shown in Fig. 6B.

Because of the use of light source 14 having a large inclination of the projecting angle to image-taking optical axis 86 in the same manner as the case of left eye 71, this method prevents light beam 84 of light source 14 from entering camera 17 directly and reduces the eyeglass reflection even if subject 70 moves the face slightly to a different orientation.

Referring back to Fig. 1, image determiner 18 evaluates quality of the eye images taken by cameras 16 and 17 and determines as to whether it is acceptable for generating an authentication code, when it obtains a result of determination indicating "the eye position is OK" from eye position determiner 33. Image determiner 18 then outputs a determination result of "image quality is OK" if it is determined acceptable for generating the authentication code, or it outputs a determination result of "image quality is NG" if it is found not suitable for generating the authentication code. On the other hand, image determiner 18 gives a direction to cameras 16 and 17 to take images again when it determines that "image quality is NG".

When memory 19 obtains the determination result of "image quality is OK" from image determiner 18, it stores the eye images of left eye 71 and right eye 72 separately.

Transmitter-receiver 20 transmits the eye images of left eye 71 and right eye 72 stored in memory 19 to authentication unit 2, and receives completion information indicating whether an authentication process is completed from authentication unit 2.

Authentication unit 2 uses the eye images taken by and obtained from image pick-up device 1 to carry out authentication to determine whether subject 70 is an individual person registered beforehand.

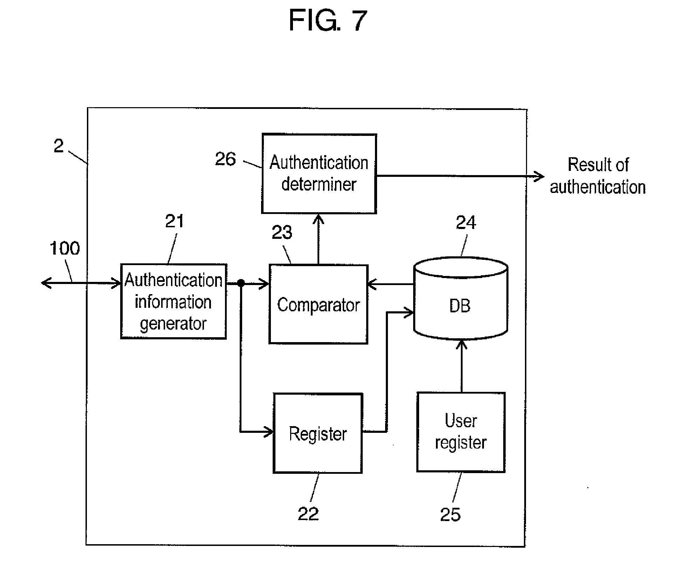

Referring now to Fig. 7, description is provided in details of authentication unit 2. Fig. 7 is a block diagram showing a structure of authentication unit 2.

Authentication unit 2 in Fig. 7 comprises authentication information generator 21 for extracting an iris pattern from the eye image obtained from image pick-up device 1 and generating authentication codes of left eye 71 and right eye 72, register 22 and comparator 23 for taking in the authentication codes generated by authentication information generator 21, authentication determiner 26 for making final authentication determination upon receiving a result of verification from comparator 23, data base 24 (hereinafter referred to as "DB 24") storing registered authentication codes of left eye 71 and right eye 72, and user register 25 in communication with DB 24.

Authentication information generator 21 generates authentication codes of left eye 71 and right eye 72 of subject 70 from the individual eye images of left eye 71 and right eye 72 obtained from image pick-up device 1.

Register 22 registers with DB 24 the authentication codes of left eye 71 and right eye 72 taken in from authentication information generator 21 in a distinctive manner as a registered left-eye authentication code and a registered right-eye authentication code of a new registrant, when they are of the new registrant.

Comparator 23 verifies the authentication code of left eye 71 generated by authentication information generator 21 in comparison with the registered authentication code of left eye 71 stored in DB 24, and computes the similarity related to left eye 71. Comparator 23 also verifies the authentication code of right eye 72 generated by authentication information generator 21 in comparison with the registered authentication code of right eye 72 stored in DB 24, and computes the similarity related to right eye 72 in the same manner. Comparator 23 computes the similarity corresponding to a hamming distance, for instance, as a degree of matching between the authentication code and the registered authentication code.

The registered authentication codes of left eye 71 and right eye 72 of subject 70 are registered and stored in advance in a distinctive manner in DB 24.

User register 25 is provided for controlling inputs of user information, and it is used when registering user information such as name of a group where a new registrant belongs, a level of authorization for access to information, age, sex, yes or no to wearing eyeglasses, yes or no to wearing contact lenses, and the like. The user information may be used for administering a threshold level for determining the authentication. It may be appropriate, for example, to set a high threshold for determining the authentication of an individual holding a high level of authorization for access to information, so as to output a more strict result of authentication.

Authentication determiner 26 determines as "matched" when the similarity of one or both of left eye 71 and right eye 72 is higher than a predetermined threshold, and outputs a result of the authentication determination that subject 70 is identified as "the original registrant". On the other hand, authentication determiner 26 determines as "not matched" when the similarities of both left eye 71 and right eye 72 are equal to or lower than the predetermined threshold, and outputs a result of the authentication determination that subject 70 is identified as "not the original registrant".

In the manner as described, authentication unit 2 obtains the eye images of right eye 72 and left eye 71 from image pick-up device 1, and generates the authentication codes individually for both right eye 72 and left eye 71. Authentication determiner 26 then determines whether or not subject 70 is an individual who has been registered beforehand based on at least one of the authentication codes for right eye 72 and left eye 71. This enables authentication unit 2 to carry out the authentication regardless of whether the registered authentication codes stored in authentication unit 2 include only one or both authentication codes for right eye 72 and left eye 71.

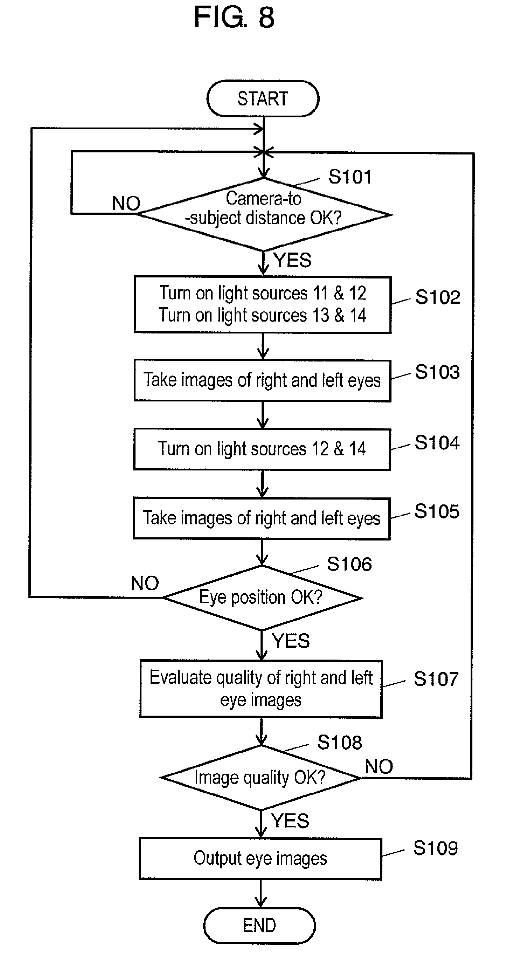

Image pick-up device 1 operates in a manner, which is described next with reference to Fig. 8. Fig. 8 is a flow chart illustrating the operation of image pick-up device 1.

As shown in Fig. 8, image pick-up device 1 has eye position determiner 33 for determining as to whether an eye position of subject 70 is within an allowable range of focal point (in the direction of Z axis) of cameras 16 and 17 (S101). That is, image pick-up device 1 determines according to an output of distance meter 32 that subject 70 is within the allowable range of focal point (YES) when, for instance, a camera-to-subject distance from the eye position is between 35cm and 45cm.

Image pick-up device 1 guides subject 70 in the forward and backward direction if the eye position of subject 70 is not inside the allowable range of focal point (NO), and it again determines whether the eye position is within the allowable range of focal point. Subject 70 can be guided by any such method as using a voice, an LED display, and the like.

When the eye position of subject 70 is within the allowable range of focal point, on the other hand, image pick-up device 1 takes images of left eye 71 and right eye 72 while switching light sources 11 to 14 with lighting controller 15.

To begin with, image pick-up device 1 lights up light sources 11, 12, 13 and 14 to provide two-side lighting (S102), and takes an image of left eye 71 with camera 16, and an image of right eye 72 with camera 17 (S103).

This method of taking the images of left eye 71 and right eye 72 of subject 70 while projecting the light from both sides can illuminate hard contact lenses uniformly and reduce shadows liable to form in the marginal areas around the lenses even when subject 70 is wearing the hard contact lenses. This method can also reduce shadows likely to form at the medial angle sides and the outer angle sides of naked eyes of subject 70.

Next, image pick-up device 1 lights up light sources 12 and 14 having a large inclination of the projecting angle to the image-taking optical axes to provide one-side lighting (S104), and takes an image of left eye 71 with camera 16, and an image of right eye 72 with camera 17 (S105).

This method of taking the images of the eyes of subject 70 while using the light sources of the large projecting angle to the image-taking optical axes can reduce eyeglass reflection even when the subject is wearing eyeglasses.

Following the above, image pick-up device 1 outputs a result of determination indicating "the eye position is OK" to image determiner 18 when it determines (YES) by eye position determiner 33 that "eyes are caught inside the angles of view" and "eye positions are within the allowable range of focal point" (S106).

On the other hand, image pick-up device 1 outputs a result of determination indicating "the eye position is NG" to image determiner 18 when it determines (NO), that is, "eyes are not caught inside the angles of view" and "eye positions are out of the allowable range of focal point" (S106), and it then returns to the step S101 to repeat taking images of the eyes of subject 70 again.

Image pick-up device 1 determines in this manner as to whether the eye positions are within the proper field for taking images.

Next, image pick-up device 1 has image determiner 18 to evaluate quality of the eye images of both left eye 71 and right eye 72 taken by cameras 16 and 17 when it obtains the determination result of "the eye position is OK" from eye position determiner 33 (S107). Image pick-up device 1 returns to the step S101 again and repeats taking images when it finds the quality of any of the images of left eye 71 and right eye 72 as being (NG) (S108).

On the other hand, image pick-up device 1 stores the eye images for both left eye 71 and right eye 72 in memory 19 in a distinctive manner when it obtains the determination result of "image quality is OK" (YES) on the both eye images (S108). At the same time, image pick-up device 1 has transmitter-receiver 20 to transmit the stored eye images for left eye 71 and right eye 72 to authentication unit 2 (S109).

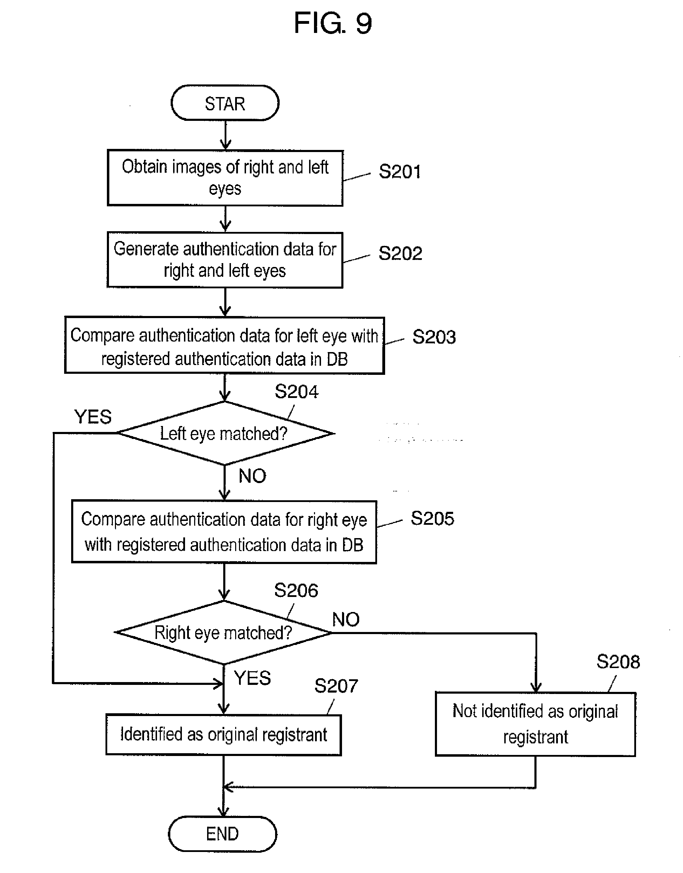

Referring to Fig. 9, description is provided next of how authentication apparatus 3 operates. Fig. 9 is a flow chart illustrating the operation of authentication apparatus 3.

As shown in Fig. 9, authentication apparatus 3 obtains the eye images of left eye 71 and right eye 72 from image pick-up device 1 (S201), and has authentication information generator 21 to generate authentication data for both left eye 71 and right eye 72 (S202).

Since authentication apparatus 3 generates the authentication data for both of left eye 71 and right eye 72, it can carry out the authentication even if subject 70 has registered only one or both of the data for left eye 71 and right eye 72 as the registered authentication data.

Next, authentication apparatus 3 has comparator 23 verify the authentication data of left eye 71 in comparison with the registered authentication data of left eye 71 stored in DB 24, and compute the similarity related to left eye 71(S203). In this step here, authentication apparatus 3 computes a degree of matching as the similarity between the authentication code and the registered authentication code.

Subsequent to the above, authentication apparatus 3 has authentication determiner 26 determine it as being "matched" when the similarity of left eye 71 is found higher than the predetermined threshold (YES) (S204), and output a result of the authentication that subject 70 is identified as "the original registrant" (S207).

On the other hand, authentication apparatus 3 determines it as being "not matched" when the similarity of left eye 71 is found equal to or lower than the predetermined threshold (NO) (S204), has comparator 23 verify the authentication data of right eye 72 in comparison with the registered authentication data of right eye 72 stored in DB 24, and compute the similarity related to right eye 72 (S205). In this step, authentication apparatus 3 computes a degree of matching as the similarity between the authentication code and the registered authentication code in the same manner as in the verification step of left eye 71. Although the description above is provided of comparator 23 to execute the comparative verification first with the authentication data for left eye 71, the comparative verification can be started with the authentication data for right eye 72.

Next, authentication apparatus 3 has authentication determiner 26 determine it as being "matched" when the similarity of right eye 72 is found higher than the predetermined threshold (YES) (S206), and output a result of the authentication that subject 70 is "the original registrant" (S207). On the other hand, authentication apparatus 3 determines it as being "not matched" when the similarity of right eye 72 is found equal to or lower than the predetermined threshold (NO) (S206), and output a result of the authentication that subject 70 is identified as "not the original registrant" (S208). This result of the authentication can be used for such applications as access control in buildings and multifamily houses, tracking of moving patterns of specific persons and the like, when adapted to door opening control, admission and access control, recording of entries and departures of certain registered persons, and the like managements.

According to the exemplary embodiment of this invention, as described, it becomes unnecessary for the subject to change the eye positions between the right eye and the left eye during the authentication since the image pick-up device is provided with the illumination lights for both the right and left eyes individually. This alleviates the inconvenience of repeating the eye positioning, and facilitates the individual subject to have images of both eyes taken at once with the optimum illumination lighting and without making an error in operating the device. It is also possible for the image pick-up device to take images of both eyes without causing any dropout even when the subject is wearing eyeglasses or hard contact lenses by virtue of reducing eyeglass reflection and shadows liable to form in the marginal areas of the hard contact lenses.

Moreover, since the image pick-up device has the lighting controller adapted to make quantities of light of the light sources individually adjustable, it can provide the lighting intensities consistent at a position of the eyes and adjust balancing in the quantities of light even when light projecting directions of the individual light sources are different in angle with respect to the image-taking optical axes. Since this structure allows adjustment of the light sources in a manner to balance the quantities of light, it can eliminate restrictions in mounting positions of the light sources, thereby allowing a reduction in size of the device when putting some contrivances into positional arrangement of the light sources.

It is also possible for this image pick-up device to equalize the lighting intensities over hard contact lenses and reduce irregularities of the lighting and shadows formed in the marginal areas by virtue of the two-side illumination even when the hard contact lenses have a large curvature.

In the exemplary embodiment of this invention, any of the lighting control function, eye position determining function, image determining function, transmitting and receiving function, authentication information generating function, comparative verification function, authentication determining function, and the like can be carried out with a hardware using an integrated circuit, etc. or a program executable by a central processing unit (CPU), digital signal processor (DSP), and the like.

### INDUSTRIAL APPLICABILITY

According to the present invention, the image pick-up device and the authentication apparatus using the same device have such an advantage as being capable of authenticating an individual subject by obtaining information of eye images of the subject, as described above. The image pick-up device is especially useful as the authentication apparatus since it can alleviate the inconvenience for the subject to make eye positioning, and help the subject to have images of both eyes taken even when the subject is wearing eyeglasses or hard contact lenses by virtue of reducing eyeglass reflection and shadows liable to form in the marginal areas of the hard contact lenses.

## Claims

1. An image pick-up device comprising:
a first image pick-up unit for taking an image of a left eye of a subject;
a second image pick-up unit for taking an image of a right eye of the subject;
a first lighting unit for illuminating the left eye of the subject;
a second lighting unit for illuminating the left eye in a direction intersecting an image-taking optical axis of the second image pick-up unit;
a third lighting unit for illuminating the right eye of the subject; and
a fourth lighting unit for illuminating the right eye in a direction intersecting an image-taking optical axis of the first image pick-up unit.

2. The image pick-up device of claim 1, wherein quantities of light of the first lighting unit through the fourth lighting unit are individually adjustable.

3. The image pick-up device of claim 2, wherein each of the first lighting unit through the fourth lighting unit comprises a plurality of light-emitting elements, and the quantities of light produced by the first lighting unit through the fourth lighting unit are adjusted individually by changing a number of the light-emitting elements to be lit.

4. The image pick-up device of claim 2, wherein each of the first lighting unit through the fourth lighting unit comprises a plurality of light-emitting elements, and the quantities of light produced by the first lighting unit through the fourth lighting unit are adjusted individually by varying a driving current supplied to each of the plurality of light-emitting elements.

5. The image pick-up device of claim 2, wherein each of the first lighting unit through the fourth lighting unit comprises a plurality of light-emitting elements, and the quantities of light produced by the first lighting unit through the fourth lighting unit are adjusted individually by a combination of changing a number of the light-emitting elements to be lit and varying a driving current supplied to each of them.

6. The image pick-up device of claim 2, wherein the quantities of light of the first lighting unit and the second lighting unit are adjusted individually according to angles of light projecting directions thereof with respect to the image-taking optical axis of the first image pick-up unit, and the quantities of light of the third lighting unit and the fourth lighting unit are adjusted individually according to angles of light projecting directions thereof with respect to the image-taking optical axis of the second image pick-up unit.

7. The image pick-up device of claim 3 further comprising a lighting controller for controlling lighting of the first lighting unit through the fourth lighting unit individually, wherein the lighting controller selectively lights up the first, second, third and fourth lighting units one after another in a predetermined lighting pattern.

8. The image pick-up device of claim 7, wherein the predetermined lighting pattern includes lighting up of any of the second lighting unit, the fourth lighting unit, both the first and the second lighting units, and both the third and the fourth lighting units.

9. The image pick-up device of claim 7, wherein the lighting controller controls individually the quantities of light produced by light sources of the first lighting unit to the fourth lighting unit in a manner to maintain consistency of the lighting intensities on both the right eye and the left eye of the subject according to a combination of the predetermined lighting patterns.

10. An authentication apparatus comprising:
the image pick-up device as recited in one of claim 1 to claim 9; and
an authentication unit for authenticating as to whether the subject is a person registered beforehand by using eye images taken on both the right eye and the left eye of the subject with the image pick-up device.

11. The authentication apparatus of claim 10, wherein the authentication unit comprises:
an authentication information generator for generating authentication information respectively representing the right eye and the left eye of the subject from the eye images taken with the image pick-up device;
a comparator for comparatively verifying the authentication information of the right eye and the left eye generated by the authentication information generator with authentication information registered beforehand; and
an authentication determiner for determining whether the subject is an individual registered beforehand by comparing a degree of matching obtained as a result of the comparative verification by the comparator with a predetermined threshold.

12. The authentication apparatus of claim 11, wherein the authentication determiner makes the determination as to whether the subject is an individual registered beforehand by using at least one of the authentication information of the right eye and the left eye.

13. The authentication apparatus of claim 11, wherein the authentication information comprises an authentication code generated by encoding an iris area of the eye image.
